# EUROPEAN PATENT APPLICATION

(11) **EP 4 616 831 A1**
(43) Date of publication of application: **17.09.2025**
(21) Application number: 24163853.5
(22) Date of filing: 15.03.2024
(51) Int. Cl.: A61F 5/56

(54) **A METHOD FOR DESIGNING AND MANUFACTURING A CUSTOM SENSORIZED ORAL APPLIANCE FOR MANAGING BRUXISM, AND A CUSTOM SENSORIZED ORAL APPLIANCE FOR MANAGING BRUXISM**

(71) Applicant: Aesyra SA, 1015 Lausanne (CH)
(72) Inventor: Letizia, Marco, 1024 Ecublens (CH); Maoddi, Pietro, 1025 Saint Sulpice (CH); Altruda, Samuele, 1024 Ecublens (CH)
(74) Representative: P&TS SA (AG, Ltd.)

(57) **Abstract**

The present invention concerns a custom sensorized oral appliance (8) for managing bruxism, comprising:
- a main body of the oral appliance, the main body comprising:
- a custom cavity (9) arranged to host a custom electronic element (20),
- the custom electronic element comprising:
- a rigid part (1),
- a force sensor (3), and
- a flexible electronic interconnection (2b) arranged for electronically connect the rigid part (1) with the force sensor (3).

The present invention concerns also a method for designing and manufacturing the oral appliance (8).

## Description

### Technical domain

The present invention concerns a method for designing and manufacturing a custom sensorized oral appliance for managing bruxism, and a custom sensorized oral appliance for managing bruxism.

### Related art

Bruxism is a condition associated with unconscious masticatory activities such as clenching, grinding or grinding teeth, which is not yet fully understood. During sleep, a person suffering from sleep bruxism typically experiences 20 to 200 episodes per night, each lasting typically 2 to 20 seconds, with jaw forces that can exceed 100 kgf. The condition is detrimental to health. Reported health complications include tooth wear, tooth shortening, prosthetic and temporomandibular joint complications (clicking, popping, etc.), masseter muscle enlargement, waking headaches and facial pain. The beauty of the smile is also affected.

Sensorized oral appliance for managing bruxism are known. An example is described by the document WO2019123232, filed by the applicant. Besides protecting teeth like a conventional bruxism oral appliance, the described oral appliance contains force sensors arranged for recording the occurrence of bruxism events.

Non-custom thermoformable oral appliances containing electronic elements are known. An example is described e.g. in the document WO2020160621A1.

In this case, the oral appliance should be moulded in hot water to adapt to irregularities and different arch sizes. However, the oral appliance remains relatively thick and the fit to the user's oral anatomy is often not perfect.

Custom designs of oral appliances are also known. In dentistry, the term "custom" typically refers to something that is made specifically for a user patient based on their unique dental anatomy and/or needs. This could include custom-made dental appliances such as crowns, bridges, dentures, orthodontic appliances, mouthguards, or oral appliances. To create custom dental appliances, dental professionals often take detailed impressions or 3D digital scans of the user's teeth and gums. These impressions or scans are then used to fabricate the oral appliance.

In this context the oral anatomy of a user comprises at least a portion of its oral cavity, including the maxillary dental arche and/or mandibular dental arche of the user. It might include also parameters extracted from the anatomy, as the curvature of dental arches, angles of teeth, distances between cusps, etc.

Solutions already exist for the computational design of custom sensorized oral appliance on the basis of collecting data of the 3D oral anatomy of the user, as e.g. described in US20190105842A1. However, this known solution is not adapted for managing bruxism. Moreover, the manufacturing process is complex and cannot be as automated as possible. Finally, the sealing of the custom sensorized oral appliance is not even discussed.

### Short disclosure of the invention

An aim of the present invention is the provision of a method for designing and manufacturing a custom sensorized oral appliance for managing bruxism, and a custom sensorized oral appliance for managing bruxism, that overcome the shortcomings and limitations of the state of the art.

Another aim of the invention is the provision of a method for designing and manufacturing a custom sensorized oral appliance for managing bruxism, allowing to maximize performances in bruxism detection.

Another aim of the invention is the provision of a method for designing and manufacturing a custom sensorized oral appliance for managing bruxism, wherein the oral appliance is as small as possible.

Another aim of the invention is the provision of a method for designing and manufacturing a custom sensorized oral appliance for managing bruxism, alternative to the state-of-the-art solutions.

An optional aim of the invention is the provision of a method for designing and manufacturing a custom sensorized oral appliance for managing bruxism, simplifying the manufacturing process.

An optional aim of the invention is the provision of a method for designing and manufacturing a custom sensorized oral appliance for managing bruxism, wherein the manufacturing process is as automated as possible.

An optional aim of the invention is the provision of a method for designing and manufacturing a custom sensorized oral appliance for managing bruxism, wherein the sealing is better than known solutions.

An optional aim of the invention is the provision of a method for designing and manufacturing a custom sensorized oral appliance for managing bruxism, wherein the oral appliance is more reliable and safer for the user.

According to the invention, these aims are attained by the object of the attached claims, and especially by a method for designing a custom sensorized oral appliance for managing bruxism of a user according to claim 1, by a method for manufacturing a custom sensorized oral appliance for managing bruxism of a user according to claim 8, and by a custom sensorized oral appliance for managing bruxism of a user according to claim 15, wherein preferred embodiments of the invention are given in the dependent claims.

The method for designing a custom sensorized oral appliance for managing bruxism of a user according to the invention, comprises the steps of:
- collect data on the 3D oral anatomy of the user, e.g. from a 3D anatomical scan, from a 3D representation of a custom oral appliance, etc.
- computationally design a main body of the oral appliance, the main body comprising:
   - a cavity arranged to host an electronic element, the cavity being customized on the basis of the data and/or based on a computationally designed electronic element,
   - computationally design the electronic element, the electronic element comprising:
      - a rigid part ,
      - a force sensor for the transduction of occlusal forces produced between teeth during bruxism, and
      - a flexible electronic interconnection arranged for electronically connect the rigid part with the force sensor,
   wherein the spatial placement in the electronic element of the rigid part, and/or of the force sensor, and/or the bending of the flexible electronic interconnection is(are) based on the data.

In one embodiment the design comprises the modification of the main body of the oral appliance.

In this context, the design is performed "computationally", i.e. with a computing module.

In one embodiment, the computing module is a machine learning-based module, including, but not limited to algorithm of 3D Pose Estimation, Object Classification, Objects Detection, Scene/Object Semantic Segmentation, 3D Geometry Synthesis/Reconstruction, Deep Learning Methods, Texture/Material Analysis, or any combination of thereof.

In this context, a machine learning-based module is a module which needs to be trained in order to learn i.e. to progressively improve a performance on a specific task. In one embodiment, the machine learning-based module is an artificial neural network, or neural network for short. It comprises a set of artificial neurons that are connected to each other to form a weighted directed graph. The neurons receive inputs from other neurons that feed into them, operate on these inputs, and transmit the results to the neurons they feed into. The edge weights denote the influence that a neuron has on those it is connected to and are computed by a process called learning which involves minimizing a loss function.

The computationally designed custom sensorized oral appliance according to the invention, including the computationally designed electronic element, comprising a rigid part, a force sensor and a flexible electronic interconnection, and including a computationally generated cavity hosting the electronic element and the bending of its flexible electronic interconnection, allows to maximize performances in bruxism detection.

The cavity of the main body of the oral appliance allows the components of the electronic elements to remain in the "right" place and to be operatively connected, in particular during bruxism events.

Since the spatial placement in the electronic element of the rigid part, and/or of the force sensor and/or the bending of the flexible electronic interconnection are based on the data on the 3D oral anatomy of the user, the space for those components is minimised, thereby rendering the oral appliance as small as possible.

In one preferred embodiment, the rigid part of the electronic element comprises components preferably not to be bent for reliability reasons, such as integrated circuits, microcontrollers, discrete electronic components, batteries, digital memories, radio-frequency antennas, coils for receiving wireless power, contacts for receiving power and/or exchanging data, sensors such as accelerometers, actuators to produce vibrations and/or sound, etc. In this context, a flexible electronic interconnection is a bendable electronic interconnection, arranged to create an electronic connection for exchanging power and/or data.

In one embodiment, the method comprises the step of computationally designing the cavity so that at least part of the cavity is in an occlusal plane and it is at least partially flexed during a bruxism event so as to activate the force sensor.

In one embodiment, the cavity comprises special structures, voids, etc.

In one embodiment, the method for designing a custom sensorized oral appliance for managing bruxism of a user comprises the step of placing the force sensor in correspondence of a cusp which produces an occlusal contact between antagonist teeth, and/or placing the rigid part around the dental arch, e.g. on the labial or lingual surfaces. Such placement maximizes the reliability of the electronics, as components are protected from occlusal forces, as well as the comfort of the appliance user.

In this context, the labial side or surface is inside the mouth, and refers to the surface facing the lips (i.e. opposite to the lingual side). The term can be used for anatomical surfaces such as teeth surfaces as well as for the surfaces of objects such as oral appliances.

In this context, the lingual side or surface is inside the mouth, refers to the surface facing the tongue (i.e. opposite to the labial side). The term can be used for anatomical surfaces such as teeth surfaces as well as for the surfaces of objects such as oral appliances.

In one embodiment, the main body comprises an insertion port, arranged for inserting the electronic element into the main body.

In this context, the insertion port is an interface used to insert the electronic element inside the oral appliance, which preferably needs to be sealed to prevent the ingress of liquids (e.g. saliva) during use and to prevent tampering with the electronic element.

In one embodiment, the insertion port has a shape and/or dimensions which are standard, i.e. independent on the oral anatomy of the user. For example, the insertion port could be a standard through opening of the main body. Its spatial position could be dependent on the oral anatomy of the user. In this case, the insertion port could be at least a part of the region of the cavity arranged to host the rigid part of the electronic element.

In another embodiment, the insertion port has a shape, dimensions and a spatial placement which depend on the oral anatomy of the user. For example, the insertion port is a volume completely included in the main body. In one embodiment, during the manufacturing the electronic element is inserted into the cavity formed between a liner and a frame, the inlet of which is an insertion port dependent on the oral anatomy of the user. In another embodiment, an additive manufacturing device as a 3D printer creates the frame, the additive manufacturing is paused to insert the electronic element, and finally the additive manufacturing is completed by sealing the oral appliance. In this case, the shape of the insertion port is not standard. In this case, the insertion port could comprise a part of the region of the cavity arranged to host the force sensor and a part of the region of the cavity hosting flexible electronic interconnections, and a part of the region of the cavity arranged to host the rigid part of the electronic element.

If an insertion port having standard shape and/or standard dimensions is present, i.e. having a shape and/or dimensions which are independent on the oral anatomy of the user (this insertion port being referred to in the following as a "standard insertion port"), the sensorized oral appliance is customized but at the same time it comprises a standard insertion port which shape and/or dimensions do not depend on the oral anatomy of the user. This allows standard electronic elements to be inserted in the insertion port of any custom oral appliance. This simplifies and renders as automated as possible the manufacturing of the oral appliance. Moreover, since the insertion port is standard, standard lids can be used to seal any custom sensorized oral appliance oral appliance. Finally, previously designed and tested methods of securely attaching and sealing the lid on the oral appliance can be used as well.

The standard geometry of the insertion port has also the advantage of decreasing the possibility of introducing defects in the seal during the sealing process and allowing a more reliable, possibly automated, inspection of the seal. These features ultimately lead to an oral appliance that is more reliable and safer for the user. Moreover, the position of the sealing components (port) can be optimized to avoid structural deformation during bruxism or during use to avoid failure and liquid penetration.

In one embodiment, the method for designing a custom sensorized oral appliance for managing bruxism of a user the comprises the step of choosing a length of the flexible electronic interconnection from a library of standard values, corresponding to available sizes of the electronic element, or computing a customized length of the flexible electronic interconnection.

In one embodiment, the method for designing a custom sensorized oral appliance for managing bruxism of a user the comprises the step of designing the region of the cavity hosting the force sensor(s) between a liner, arranged to be in contact with the teeth of the arch on which the oral appliance is worn, and a frame arranged to be in contact with the teeth of the antagonist dental arch.

In this context, a linear is a part of the oral appliance in contact with the dental arch on which the oral appliance is worn. The liner is typically made of a softer material to provide higher deformation of the cavity and/or a better grip on the teeth.

In this context, a frame is a part of the oral appliance that faces the dental arch antagonist to the one on which the appliance is worn. The frame is typically made of a harder and stiffer material in order to resist mechanical wear and avoid a "chewy" sensation to the user of the appliance.

In one embodiment, the electronic element is sandwiched between the liner and the frame.

In one embodiment, the sealing of the oral appliance is obtained by bonding the liner and the frame together. In this embodiment, a lid can be present or not.

In one embodiment, the method for designing a custom sensorized oral appliance for managing bruxism of a user the comprises the step of designing the region of the cavity hosting the force sensor, so that it is entirely contained within the liner or the frame.

In one embodiment, the force sensor is compressible, in order to transduce the force signal (e.g. capacitive, piezoresistive, piezoelectric, ...).

In one embodiment, the method for designing a custom sensorized oral appliance for managing bruxism of a user the comprises the step of keeping into account the geometry of a teeth of the dental arch on which the oral appliance is to be worn, in order to obtain a shape that provides retention on such teeth, e.g. by wrapping the tooth lingual and labial surfaces at a sufficient height.

In one embodiment, the method for designing a custom sensorized oral appliance for managing bruxism of a user that comprises the step of designing the oral appliance so that its shape grips on the teeth of the dental arch on which it is to be worn.

In one embodiment, the rigid part is a first rigid part, and the electronic element comprises a second rigid part and a second flexible electronic interconnection arranged for electronically connect the first rigid part with the second rigid part.

The method for manufacturing a custom and sensorized oral appliance for managing bruxism of a user according to the invention comprises the steps of:
- designing the oral appliance with the method for designing a custom sensorized oral appliance for managing bruxism of a user according to the invention,
- manufacturing the main body of the oral appliance and the electronic element.

In one embodiment, the method for manufacturing a custom and sensorized oral appliance comprises the step of inserting the electronic element into the main body via the insertion port.

In one embodiment, the method for manufacturing a custom and sensorized oral appliance comprises the step of sliding the electronic element into its rest operating position in the cavity.

In one embodiment, the insertion port comprises a hole and the flexible electronic interconnection is inserted in its receiving cavity region through this hole.

In one embodiment, the method for manufacturing a custom and sensorized oral appliance comprises the step of the deformation (e.g. by elongation) of the flexible electronic interconnection of the electronic element during its insertion and/or sliding.

In one embodiment, the force sensor and the rigid part are inserted in their respective receiving cavity regions through the insertion port.

In one embodiment, the manufacturing is performed with additive processes, for example 3D printing, selective laser sintering, stereolithography, etc.

In one embodiment, the manufacturing is performed with subtractive processes, for example CNC machining, electro-discharge machining, etc.

In one embodiment, the method for manufacturing a custom and sensorized oral appliance for managing bruxism of a user comprises the step of changing the stiffness of the cavity using the same material, therefore changing the response of the force sensor, e.g. to the applied force.

In one embodiment, the method for manufacturing a custom and sensorized oral appliance for managing bruxism of a user comprises the step of closing the insertion port with a lid.

In one embodiment, the method for manufacturing a custom and sensorized oral appliance for managing bruxism of a user comprises the step of sealing the oral appliance once the lid has been attached and/or the step of sealing the liner and the frame together, e.g. by vapor smoothing, chemical smoothing, abrasive methods, heat polishing, filling materials, mechanical polishing, chemical vapour deposition processes, etc.

In one embodiment, the insertion port is a first port and the method for manufacturing a custom and sensorized oral appliance for managing bruxism of a user comprises the step of recovering an end of the flexible electronic interconnection from a second insertion port opposite to the first insertion port.

In one embodiment, the method for manufacturing a custom and sensorized oral appliance for managing bruxism of a user comprises the step of connecting the end of the flexible electronic interconnection to a second rigid part of the electronic element, e.g. via a connector, thus completing the electronic connection. The assembly of the oral appliance can be completed by inserting a second force sensor and a second rigid part in their respective receiving cavity regions through the second insertion port, which can be finally sealed closed, e.g. by the second lid.

In one embodiment, the sealing between the lid and the oral appliance can be obtained using an O-ring and/or mechanical snap-in features on the lid.

In one embodiment, the sealing can be obtained by attaching the lid to the oral appliance, e.g. through the use of an adhesive, for example cyanoacrylate glue, epoxy glue, hybrid glue, hotmelt, etc.

In one embodiment, the lid is a pre-integrated lid, for example by injection over-molding of plastic, with the electronic element.

In one embodiment, the lid is overmolded on a rigid part of the electronic element, in such a way that electronic contacts for exchanging power and/or data are integrated in the lid.

In one embodiment, the entire outer surface of the lid is metallic and is used as an electronic contact for exchanging power and/or data.

In one embodiment, power and/or data can be wirelessly transferred to the oral appliance.

In one embodiment, the cavity is obtained between two materials of different stiffness.

In one embodiment, the cavity is obtained within a homogenous material.

In one embodiment, part or the entirety of the electronic element is partially or entirely encapsulated in a sealing material, e.g. parylene-coated, injection over-molded with plastic, etc.

In one embodiment, the oral appliance is made of one plastic material or multiple plastic materials.

The custom sensorized oral appliance for managing bruxism of a user according to the invention comprises:
- a main body of the oral appliance, the main body comprising:
   - a customised cavity arranged to host a customised electronic element,
   - the customised electronic element comprising:
      - a rigid part,
      - a force sensor, and
      - a flexible electronic interconnection arranged for electronically connect the rigid part with the force sensor.

In this context, an electronic element is custom or customized, if the spatial placement in the electronic element of the rigid part, and/or of the force sensor and/or the bending of the flexible electronic interconnection is(are) based on the data on the 3D oral anatomy of the user.

### Short description of the drawings

Exemplar embodiments of the invention are disclosed in the description and illustrated by the drawings in which:
Figure 1 illustrates schematically a front view of the electronic element according to one embodiment of the invention.
Figure 2 illustrates schematically a perspective view of a model of the electronic element and of a part of the 3D oral anatomy of the user according to one embodiment of the invention.
Figure 3 illustrates schematically a cross-section view of a model of a tooth, of its antagonist tooth and of the electronic element according to one embodiment of the invention.
Figure 4A illustrates schematically a perspective view of the oral appliance worn by a user, according to one embodiment of the invention.
Figure 4B illustrates schematically the external aspect of the oral appliance of Figure 4A, worn by a user.
Figure 5 illustrates schematically a cross-section view of a model of a tooth, of its antagonist tooth and of the oral appliance according to one embodiment of the invention.
Figure 6 illustrates schematically a cross-section view of a model of a tooth, of its antagonist tooth and of the oral appliance according to one embodiment of the invention.
Figure 7A illustrates schematically a perspective view of the oral appliance worn by a user, according to one embodiment of the invention.
Figure 7B illustrates schematically the external aspect of the oral appliance of Figure 7A, worn by a user.
Figure 8A illustrates schematically a perspective view of the oral appliance worn by a user, according to one embodiment of the invention.
Figure 8B illustrates schematically the external aspect of the oral appliance of Figure 8A, worn by a user.
Figure 9 illustrates schematically a partially exploded perspective view of the oral appliance according to one embodiment of the invention.
Figure 10 illustrates the oral appliance of Figure 9 with another perspective view.
Figure 11 illustrates schematically a partially exploded perspective view of the oral appliance according to one embodiment of the invention.
Figure 12 illustrates schematically a cross-section view of a portion of the oral appliance according to one embodiment of the invention.
Figure 13 illustrates schematically a cross-section view of a portion of the oral appliance according to one embodiment of the invention.
Figure 14 illustrates schematically a perspective view of a portion of the oral appliance according to one embodiment of the invention.
Figure 15 illustrates schematically a cross-section view of a portion of the oral appliance according to one embodiment of the invention.

### Examples of embodiments of the present invention

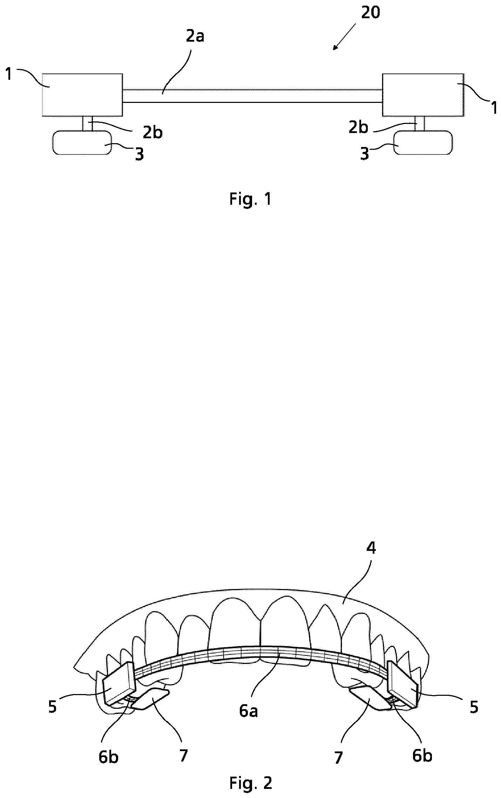

The oral appliance 8 according to the invention comprises an electronic element 20, an embodiment of which is illustrated in Figure 1.

The electronic element 20 comprises at least one rigid part 1, two in the embodiment of Figure 1. Each rigid part 1 is not-bendable and typically containing components preferably not to be bent for reliability reasons, such as integrated circuits, microcontrollers, discrete electronic components, batteries, digital memories, radio-frequency antennas, coils for receiving wireless power, contacts for receiving power and/or exchanging data, sensors such as accelerometers, actuators to produce vibrations and/or sound, etc.

The electronic element 20 comprises flexible electronic interconnection 2a between the rigid parts 1. The flexible electronic interconnection 2a cannot be present in embodiments where only one rigid part 1 is included in the electronic module 20. The flexible electronic interconnection 2a is bendable and can include some components.

The electronic element 20 also includes at least one force sensor 3, two in the embodiment of Figure 1, connected to the rigid parts 1 via a flexible electronic interconnection 2b. Also, the flexible electronic interconnection 2b is bendable and can include some components.

In one embodiment, the force sensor 3 is compressible, in order to transduce the force signal, e.g. capacitive, piezoresistive, piezoelectric, etc.

In one preferred embodiment, if the electronic element 20 is not yet mounted in the oral appliance, the flexible electronic interconnection 2a is substantially perpendicular to the flexible electronic interconnections 2b, the rigid part 1 is substantially coplanar with the sensor 3, and the flexible electronic interconnections 2a, 2b are linear.

Part or the entirety of the electronic element 20 can be partially or entirely encapsulated in a sealing material, e.g. parylene-coated, injection over-molded with plastic.

In one embodiment, the spatial placement of the rigid parts 1 and of the force sensors 3, and the bending of the flexible electronic interconnections 2a, 2b, is computationally designed using models of the electronic element 5, 6a, 6b, 7 and of the oral anatomy of the user 4, in particular including a model of the 3D oral anatomy of the user, e.g. collected by an anatomical scan, including 3D models of the maxillary and mandibular dental arches. In Figure 2, only the maxillary arch is shown for clarity.

In one embodiment, once the electronic element 20 is mounted in the oral appliance, the rigid part 1 forms an angle with the sensor 3. This angle is obtained by bending the flexible electronic interconnections 2b.

In one embodiment, once the electronic element 20 is mounted in the oral appliance, flexible electronic interconnection 2a is curved. This is obtained by bending the flexible electronic interconnection 2a.

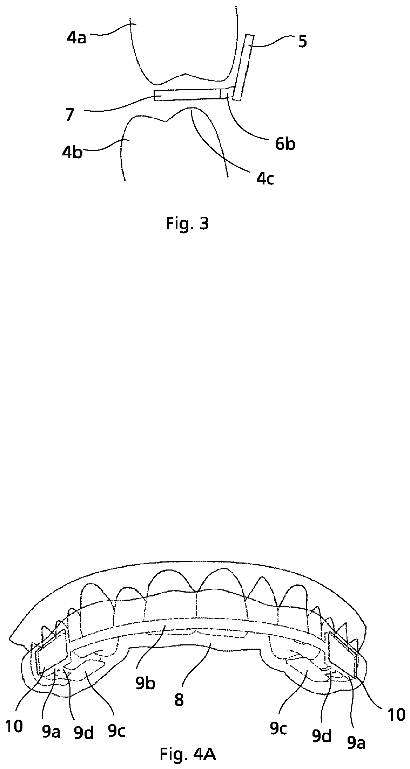

In particular, as visible in Figure 3, the force sensor's models 7 are placed in correspondence of the cusps 4c which produce an occlusal contact between antagonist teeth 4a, 4b, while the rigid part models 5 are placed around the dental arch, on the labial or lingual surfaces. Such placement maximizes the reliability of the electronics, as components are protected from occlusal forces, as well as the comfort of the user.

The bending of the flexible connection models 6a between the rigid parts is calculated to marry such surfaces, while the bending of the flexible electronic interconnection models 6b between the force sensors and the rigid parts of the electronic is computationally designed to match the positions of such elements.

The position of models 5, 7 can be computationally adjusted in order to respect constraints relative to the oral anatomy 4a, 4b, for example to respect minimum or maximum distances, as well as to respect constraints relative to the flexible electronic interconnection models 6a, 6b, for example a prescribed connection length, a minimum bending radius, etc.

In some embodiments, an optimal length of flexible electronic interconnection models 6a, 6b can be chosen from a library of standard values, corresponding to available sizes of the electronic element 20, or calculated a custom length for such interconnections, corresponding to the length at which a fixed-size electronic substrate needs to be cut to adapt to the electronic modules' distance in the oral appliance 8.

The main body of the oral appliance 8 is also computationally designed, by featuring a cavity 9 for the placement of the electronic element 20. In one embodiment, the cavity 9 is designed based on the design of the electronic element 20.

In one embodiment, the cavity 9 comprises a region 9a for the placement of the rigid parts, regions 9b, 9d for the placement of the flexible electronic interconnections and a region 9c for the placement of the force sensor 9c.

The cavity 9 allows the components of the electronic module 20 to remain in the right place and operatively connected, in particular during bruxism events.

The cavity 9 allows also the electronic element 20, comprising the sensor 3, to slide during the mounting phase into the rest operating position.

The cavity 9 allows also the electronic element 20 to be host in place during the rest operating position and comply with possible deformation during the sensor compression.

The cavity 9 allows also the electronic element 20 to deform to comply with elongation needed during the mounting phase.

In one embodiment, the main body of the oral appliance 8 includes at least one insertion port, which in one embodiment is an opening to be used for the assembly of the electronic element 20 into the oral appliance 8.

In one embodiment, the insertion port is closed by a lid 10, e.g. during the final assembly of the oral appliance 8.

In one embodiment, the lid 10 seals at least one end of the cavity 9.

In one embodiment, the lid 10 holds a (press-fit) O-ring to seal the closure.

In one embodiment, the lid 10 a gap with the oral appliance that is filled with a sealing agent.

In one embodiment, the lid 10 exposes an optical sensor towards the buccal side. In one embodiment, the optical sensor is a pulse oximeter towards the buccal side to measure oxygen saturation in the buccal area.

In one embodiment, the design of the main body of the oral appliance 8 also keeps into account the geometry of the teeth 4a of the dental arch on which the oral appliance 8 is to be worn, in order to obtain a shape that provides retention on such teeth, e.g. by wrapping the tooth lingual and labial surfaces at a sufficient height.

In one embodiment, the oral appliance 8 is manufactured with an additive process, for example 3D printing, selective laser sintering, stereolithography, etc.

In one embodiment, the oral appliance 8 is manufactured with a subtractive process, for example CNC machining, electro-discharge machining, etc.

The oral appliance 8 features cavities 9c for the force sensors. The force sensors can be compressed upon force exerted between antagonist teeth 11a, 11b. Such compression is optimal as a result of placement in correspondence of the cusps 11c and of the designed stiffness reduction around the cavity 9c resulting from the material choice and thickness.

In one embodiment, the oral appliance 8 also has a shape that grips on the teeth 11a of the dental arch on which it is to be worn.

In one embodiment, the oral appliance 8 comprises geometrical structures to change the stiffness of the cavities using the same material, therefore changing the response of the force sensors to the applied force.

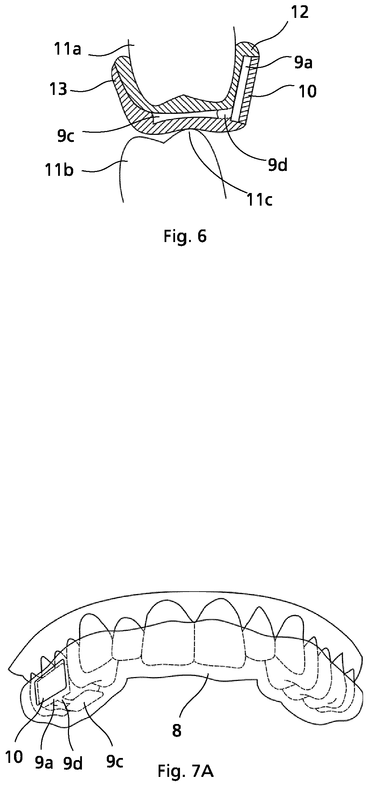

In one embodiment, the cavity 9 is obtained between two materials of different stiffness rather than within a homogenous material, as illustrated e.g. in Figure 6.

In one embodiment, the region 9c of the cavity hosting the force sensors is formed between a liner 12, in contact with the teeth 11a of the arch on which the oral appliance is worn, and a frame 13 which contact the teeth 11b of the antagonist dental arch.

The liner 12 is typically made of a softer material in order to provide higher deformation of the cavity 9 and better grip to the teeth, while the frame 13 is typically made of a harder and stiffer material in order to resist mechanical wear and avoid a "chewy" sensation to the user of the oral appliance.

In the case in which the cavity 9 is obtained between the liner 12 and the frame 13, the lid 10 can also be absent, the electronic element 20 being sandwiched between the liner 12 and the frame 13. The sealing of the oral appliance 8 can be obtained by bonding the liner 12 and the frame 13 together.

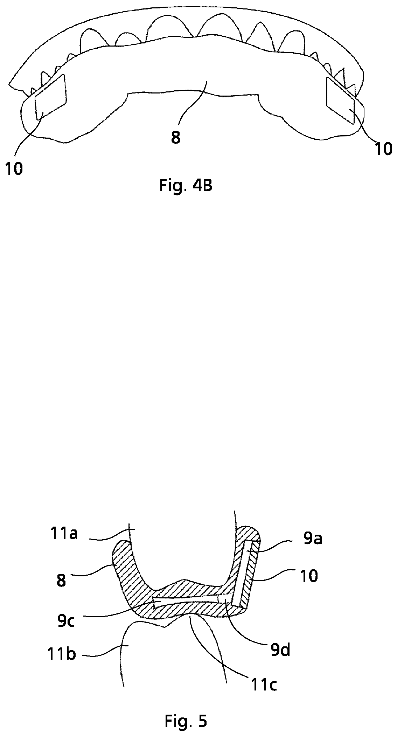

In one embodiment, the cavity 8 is entirely contained within the liner 12 or in the frame 13, rather than formed between the two, i.e. adding a liner 12 or a frame 13 to the oral appliance represented in Figure 5.

In one embodiment, the cavity region 9a containing the flexible electronic interconnections between rigid parts is completely buried in the material, as illustrated in Figure 4.

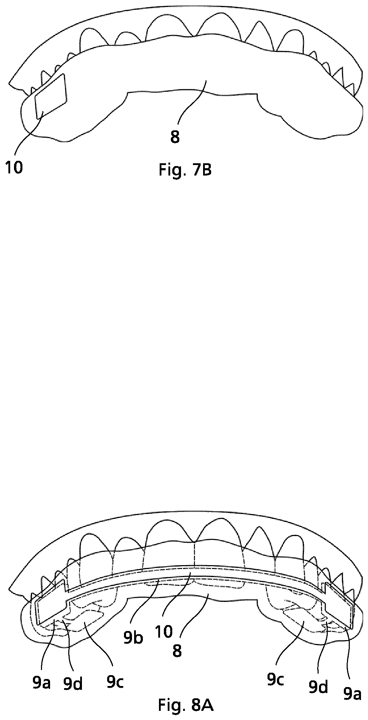

In one embodiment, the cavity region 9a containing the flexible electronic interconnections between rigid parts is absent in reason of there being only one rigid part, as illustrated in Figure 7.

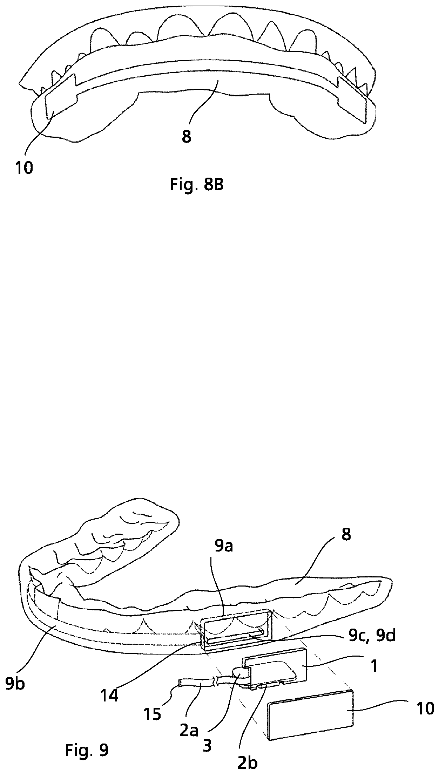

In one embodiment, the cavity region 9a containing the flexible electronic interconnections between rigid parts is shaped as to present an opening in correspondence of the appliance surface as illustrated in Figure 8, effectively becoming part of the insertion ports for the electronic element 20, which is sealed closed by lids 10 during the final assembly of the device.

The manufacturing of the oral appliance 8, and in particular its assembly, can be performed in several ways.

In one embodiment, illustrated in Figure 9, the flexible electronic interconnection 2a stemming from a first rigid part 1 is initially open-ended, terminating in a connector 15. Such flexible electronic interconnection 2a is inserted in its receiving cavity region 9b of the cavity 9 through a hole 14 connecting regions 9a and 9b of the cavity 9, while a first force sensor 3 and the first rigid part 1 are inserted in their respective receiving cavity regions 9c and 9a through a first insertion port represented by the open face of cavity 9a. Such insertion port can be sealed closed by a lid 10.

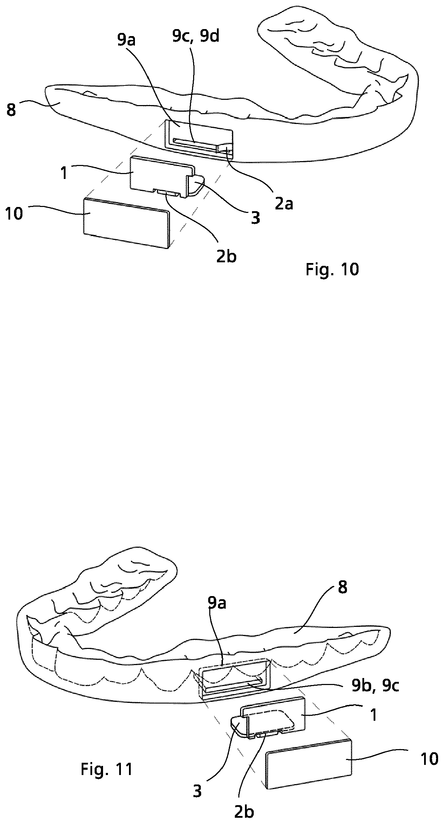

In one embodiment, illustrated in Figure 10, the assembly continues by recovering the open-ended flexible electronic interconnection 2a from the opposite side of the oral appliance 8. Such flexible electronic interconnection 2a can be then connected to a second rigid part 1 of the electronic element via the connector 15, thus completing the electronic connection.

The assembly of the oral appliance 8 can completed by inserting the second force sensor 3 and the second rigid part 1 in their respective receiving cavity regions 9c and 9a through the second insertion port represented by the open face of cavity 9a, which can be finally sealed closed by the second lid 10.

In another embodiment, illustrated in Figure 11, the oral appliance is devoid of the flexible electronic interconnection 2a and the single force sensor 3 and the rigid part 1 are inserted in their respective receiving cavity regions 9c and 9a through the insertion port represented by the open face of cavity 9a, which can be finally sealed closed by the second lid 10.

In one embodiment the oral appliance 8 comprises insertion port(s) and possible lid(s), the shape and/or dimensions of which are standard, i.e. they do not depend on the oral anatomy of the user. This present several advantages:
- standard electronic element(s) 20 can be inserted in the insertion ports of any custom oral appliance 8,
- standard lid(s) 10 can be used to seal any custom oral appliance 8,
- previously designed and tested methods of securely attaching and sealing the lid 10 on the oral appliance 8, can be used such as screws, press-fit or snap-in mechanical joints or (automatically applied) adhesives, possibly in combination with gaskets or O-rings to improve water-proofness of the sealing. Any combination of the previously designed and tested methods of securely attaching and sealing the lid 10 on the oral appliance 8 can be used as well.

The small size (the size ranging from hundreds of micrometres to few centimetres) and standard geometry of the standard insertion port(s) (and of the possible lid(s)) also have the advantages of decreasing the possibility of introducing defects in the seal during the sealing process and allowing a more reliable, possibly automated, inspection of the seal. These features ultimately lead to an oral appliance 8 that is more reliable and safer for the user.

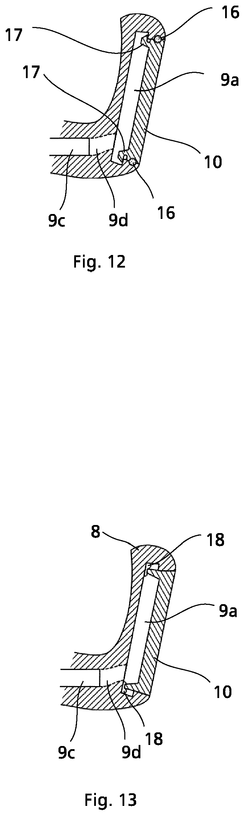

In one embodiment, illustrated in Figure 12, the sealing between the lid 10 and the oral appliance 8 can be obtained using a sealing joint 16, as a gasket, or an O-ring, and mechanical snap-in features 17 on the lid 10.

In other embodiment, illustrated in Figure 13, between the lid 10 and the oral appliance 8 can be obtained by attaching the lid 10 to the oral appliance 8 through an adhesive or gluing substance 18, for example cyanoacrylate glue, epoxy glue, hybrid glue, hotmelt, etc.

Another advantage stemming from the standard insertion port(s) and lid(s) is the possibility to use lid pre-integrated with the electronic element, for example by injection over-molding of plastic.

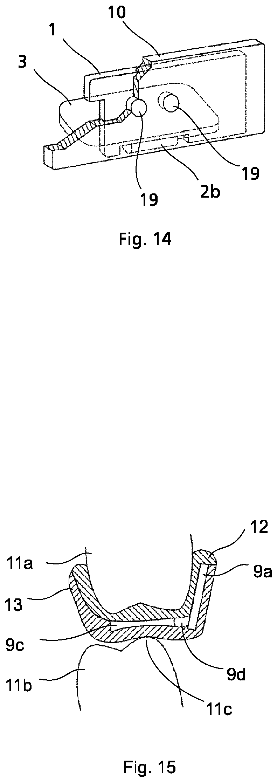

In one embodiment, illustrated in Figure 14, the lid 10 is made of plastic that is overmolded on a rigid part 1 of the electronic element, in such a way that electronic contacts 19 for exchanging power (for recharging the battery) and/or data are integrated in the lid 10. In other embodiments, the transfer of power and/or data is wirelessly performed.

In one embodiment, the oral appliance 8 is sealed, e.g. once the lid has been placed, and/or the liner and frame are sealed together. The sealing can be performed, e.g. by vapor smoothing, chemical smoothing, abrasive methods, heat polishing, filling materials, mechanical polishing, chemical vapour deposition processes, etc.

In one embodiment, the oral appliance 8 is sealed in correspondence of a computationally designed region. In one embodiment, the computationally designed region is at least partially extended along the interface between the liner and the frame, illustrated in Figure 6.

In one embodiment, the computationally designed region is reached by a sealant agent once the electronic module is inserted in the cavity 9.

In one embodiment, the computationally designed region is reached by a sealing agent via an opening in the appliance body. The sealing agent can be acrylic or UV curable or epoxy based or based on the same material of the oral appliance 8.

In one embodiment, the insertion port has a shape, dimensions and a spatial placement which depend on the oral anatomy of the user. For example, the insertion port is a volume completely included in the main body. In one embodiment, illustrated in Figure 15, the electronic element during the manufacturing is inserted into the cavity formed between a liner 12 and a frame 13, the inlet of which is an insertion port dependent on the oral anatomy of the user.

In another embodiment, an additive manufacturing device as a 3D printer creates the frame 12, the additive manufacturing is paused to insert the electronic element, and finally the additive manufacturing is completed by sealing the oral appliance. In this case, the shape and the dimensions of the insertion port are not standard.

### Reference signs used in the figures

- 1: Rigid part
- 2a, 2b: Flexible electronic interconnections
- 3: Sensors
- 4: Model of the user oral anatomy
- 4a: Model of a tooth
- 4b: Model of the antagonist tooth
- 4c: Position of the antagonist tooth cusp
- 5: Model of the rigid part
- 6, 6a, 6b: Model of flexible electronic interconnections
- 7: Model of the sensor
- 8: Oral appliance
- 9a: Region of the cavity hosting the rigid part
- 9b: Region of the cavity hosting flexible electronic interconnections between rigid parts
- 9c: Region of the cavity hosting the sensor
- 9d: Region of the cavity hosting flexible electronic interconnections
- 10: Lid
- 11a: Tooth of the dental arch on which the appliance is worn
- 11b: Antagonist tooth
- 11c: Cusp of the antagonist tooth that compresses the cavity
- 12: Liner
- 13: Frame
- 14: Hole
- 15: Electronic connector
- 16: Sealing joint
- 17: Snap-in feature of the lid
- 18: Adhesive
- 19: Electronic contacts
- 20: Electronic element

## Claims

1. A method for designing a custom sensorized oral appliance (8) for managing bruxism of a user, comprising the steps of:
- collect data on the 3D oral anatomy of the user,
- computationally design a main body of the oral appliance (8), the main body comprising:
- a cavity (9) arranged to host an electronic element (20), the cavity being customized on the basis of the data, and/or on the basis of a computationally designed electronic element,
- computationally design the electronic element (20), the electronic element comprising:
- a rigid part (1, 5),
- a force sensor (3, 7) for the transduction of occlusal forces produced between teeth during bruxism, and
- a flexible electronic interconnection (2b, 6b) arranged for electronically connect the rigid part (1, 5) with the force sensor (3, 7),
wherein the spatial placement in the electronic element (20) of the rigid part (1, 5), and/or of the force sensor (3, 7), and/or the bending of the flexible electronic interconnection (2b, 6b) is(are) based on the data.

2. The method of claim 1, comprising the step of computationally designing the cavity (9) so that at least part of the cavity (9) is in an occlusal plane and it is at least partially flexed during a bruxism event so as to activate the force sensor (3, 7).

3. The method of one of claims 1 or 2, comprising the step of placing the sensor (3, 7) in correspondence of a cusp (4c) which produce an occlusal contact between antagonist teeth (11b), and/or placing the rigid part (1, 5) around the dental arch, e.g. on the labial or lingual surfaces.

4. The method of one of claims 1 to 3, wherein the main body comprises an insertion port, for inserting the electronic element (20) into the main body.

5. The method of one claim 4, wherein the insertion port is a standard insertion port, having a shape and/or dimensions which are independent on the oral anatomy of the user.

6. The method of one of claims 1 to 5, comprising the step of designing the region (9c) of the cavity (9) hosting the force sensor (3, 7) between a liner (12), arranged to be in contact with the teeth of the arch on which the oral appliance is worn, and a frame (13) arranged to be in contact with the teeth of the antagonist dental arch.

7. The method of one of claims 1 to 6, wherein the rigid part (1, 5) is a first rigid part, and the electronic element (20) comprises a second rigid part (1, 5) and a second flexible electronic interconnection (2b, 6b) arranged for electronically connect the first rigid part with the second rigid part.

8. A method for manufacturing a custom and sensorized oral appliance (8) for managing bruxism of a user, comprising the steps of:
- designing the oral appliance (8) with the method for designing a custom sensorized oral appliance for managing bruxism of a user according to one of the claims 1 to 7,
- manufacturing the main body of the oral appliance (8) and the electronic element (20),.

9. The method of claim 8, comprising the step of inserting the electronic element (20) into the main body via an insertion port

10. The method of claim 9, wherein the insertion port comprises a hole (14) and the flexible electronic interconnection (9b) is inserted in its receiving cavity region (9b) through this hole (14).

11. The method of one of claims 9 to 10, comprising the step of sliding the electronic element (20) into its rest operating position in the cavity (9) and /or the step of the deformation of the flexible electronic interconnection (9b) of the electronic element (20) during its insertion and/or sliding.

12. The method of one of claims 9 to 11, comprising the step of closing the insertion port with a lid (10).

13. The method of one of claims 8 to 12, comprising the step of sealing the oral appliance (8) once the lid (10) has been attached and/or sealing a liner (12) and a frame (13) together.

14. The method of one of claims 9 to 13, wherein the insertion port is a first port, the method comprising the step of recovering an end of the flexible electronic interconnection from a second insertion port opposite to the first insertion port.

15. Custom sensorized oral appliance (8) for managing bruxism of a user, comprising:
- a main body of the oral appliance, the main body comprising:
- a custom cavity (9) arranged to host a custom electronic element (20),
- the custom electronic element comprising:
- a rigid part (1),
- a force sensor (3), and
- a flexible electronic interconnection (2b) arranged for electronically connect the rigid part (1) with the force sensor (3).
